(19) Europäisches Patentamt
European Patent Office
Office européen des brevets

(11) Publication number: **0 080 942 B1**

(12) **EUROPEAN PATENT SPECIFICATION**

(45) Date of publication of patent specification: **26.02.86**

(51) Int. Cl.$^4$: **C 07 D 205/08** // C07D499/46

(21) Application number: **82402149.7**

(22) Date of filing: **26.11.82**

(54) **Novel intermediates and process for preparing (S)-3-acylamino-4-substituted-2-azetidinones.**

(30) Priority: **30.11.81 US 325781**

(43) Date of publication of application: **08.06.83 Bulletin 83/23**

(45) Publication of the grant of the patent: **26.02.86 Bulletin 86/09**

(84) Designated Contracting States: **AT BE CH DE FR GB IT LI LU NL SE**

(56) References cited:
**EP-A-0 060 120**
**US-A-4 158 004**

**MONATSHEFTE FÜR CHEMIE, vol. 3, no. 98, 1967, Springer-Verlag, pages 755-762, New York (USA); H. STETTER et al.: "Über Verbindungen mit Urotropin-Struktur, 37. Mitt.: Eine neue Methode zur Herstellung von Hydroxy-ketonen und Diketonen sowie ihre Anwendung auf die Synthese des 2,4-Dioxa-adamantan-Ringsystems".**

(73) Proprietor: **E.R. Squibb & Sons, Inc.
Lawrenceville-Princeton Road
Princeton, N.J. 08540 (US)**

(72) Inventor: **Mueller, Richard H.
7 Pin Oak Drive
Lawrenceville New Jersey (US)**
Inventor: **Cimarusti, Christopher M.
278 Wargo Road
Pennington New Jersey (US)**
Inventor: **Kissick, Thomas P.
371 Ridgeviex Road
Princeton New Jersey (US)**

(74) Representative: **Maiffret, Bernard et al
Law Offices of William J. Rezac 49, avenue
Franklin D. Roosevelt
F-75008 Paris (FR)**

(56) References cited:

**THE JOURNAL OF ORGANIC CHEMISTRY, vol. 38, no. 5, 9th March 1973, pages 940-943; J.C. SHEEHAN et al.: "The removal and displacement of the thiazolidine ring of penicillin. I. 3-Acylaminoazetidinone and 3-Acylamino-4-phenylthioazetidinone".**

**J.C.S. Chem. Comm., 1980, pg. 736-737, Kobayashi et al.**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**EP 0 080 942 B1**

**Description**

Compounds having the formula

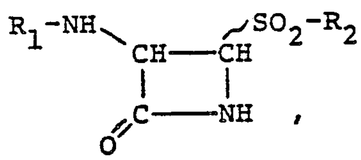

I

wherein $R_1$ is one of the simple acyl groups phenylacetyl or phenoxyacetyl, can be treated with a Grignard reagent having the formula

$$R_3\text{—Mg-X,} \qquad II$$

to yield the corresponding compounds having the formulas

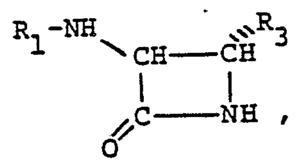

IIIa

and

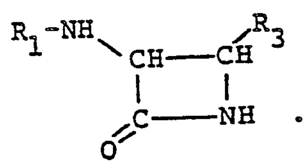

IIIb

In the above formulas, and throughout the specification, the symbols are as defined below.

$R_1$ is phenylacetyl or phenoxyacetyl;

$R_2$ is a branched chain alkyl group, phenyl or phenyl substituted with 1, 2 or 3 alkyl (of 1 to 4 carbon atoms) or alkoxy (of 1 to 4 carbon atoms) groups, or norbornyl;

$R_3$ is straight or branched chain $C_{1-10}$ alkyl, $C_{2-10}$ alken-1-yl or alkyn-1-yl, 2-phenylethenyl, 2-phenylethynyl, aryl or arylalkyl wherein aryl is phenyl or phenyl substituted with 1, 2 or 3 alkyl (of 1 to 4 carbon atoms) or alkoxy (of 1 to 4 carbon atoms) groups, and

$X_1$ is bromine or chlorine, preferably chlorine.

Listed below are definitions of various terms used herein. These definitions apply to the terms as they are used throughout the specification (unless they are otherwise limited in specific instances) either individually or as part of a larger group.

The terms "alken-1-yl" and "alkyn-1-yl" refer to both straight and branched chain groups.

Exemplary of the branched chain alkyl groups representing $R_2$ are isopropyl and *t*-butyl.

These compounds of formula I wherein $R_2$ is norbornyl are novel compounds, and as such, they form an integral part of this invention. Compounds of formula I are obtained from the well known fermentation products penicillin G (benzyl penicillin), penicillin V, or 6-APA(6-aminopenicillanic acid), using any one of several reaction sequences.

One such reaction sequence comprises conversion of pen G or pen V to the corresponding sulfoxide ester (see, for example, *Cephalosporins and Penicillins, Chemistry and Biology,* E. H. Flynn, editor., Academic Press, 1972), followed by rearrangement, in-situ norbornylene trapping and conjugation to give a compound having the formula

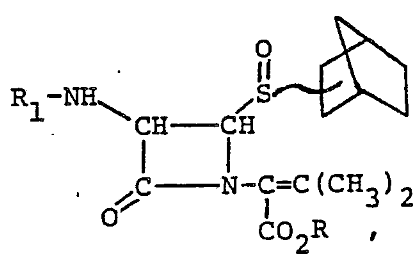

IV

wherein the "$CO_2R$" group is an esterified carboxyl group, such as an alkyl ester or trialkylsilyl ester. Subsquent oxidation and cleavage by treatment with an acid yields the corresponding (3R-*cis*)-3-acylamino-4-norbornylsulfonyl-2-azetidinone having the formula

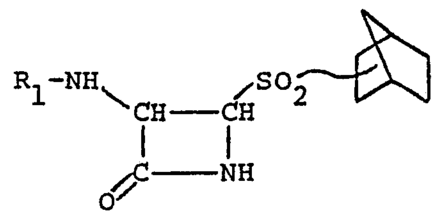

V

as a mixture of diastereomers. The mixture can be separated using conventional techniques or preferably, will be used in the next step of the process of this invention as a mixture. Compounds of formula V form an integral part of this invention.

Treatment of the above (3R-*cis*)-3-acylamino-4-norbornylsulfonyl-2-azetidinone with the appropriate mercaptan having the formula $R_2$—SH in the presence of a base yields the corresponding compound having the formula

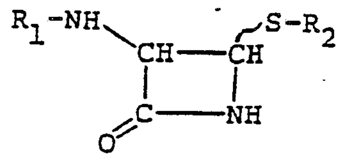

VI

(see *J. Org. Chem., 38:*940 (1973)), which can be oxidized to yield the desired starting material of formula I.

Alternatively, pen G or pen V can be converted to 6-APA, which can be converted to a compound having the formula

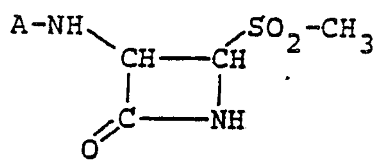

VII

wherein A is a conventional amino-protecting group (see *J. Chem. Soc.,* Perkin I, 562 (1975)). Treatment of a compound of formula VII with sodium aryl sulfinate in the presence of tetra-n-butyl ammonium bromide under phase transfer conditions gives the corresponding compound having the formula

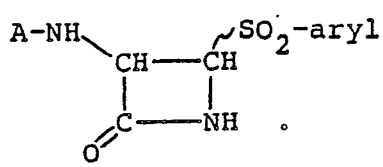

VIII

Deprotection of a compound of formula VIII followed by acylation yields the desired starting material of formula I (wherein $R_2$ is aryl) as a mixture of the *cis* and *trans* isomers, which are separable by fractional crystallization and/or column chromatography.

The conversion of a compound of formula I to a mixture of compounds of formulas IIIa and IIIb is accomplished by treating a compound of formula I with a Grignard reagent of formula II, preferably in the presence of a Lewis acid. Magnesium chloride is the preferred Lewis acid. The converstion is accomplished most efficiently using an excess of Grignard reagent, preferably three (3) molar equivalents, and most preferably, four (4) or five (5) molar equivalents. Preferably about four (4) to six (6) molar equivalents of Lewis acid are used.

A mixture of compounds of formulas IIIa and IIIb can be separated using art-recognized techniques such as column chromatography and fractional crystallization.

The compounds of formulas IIIa and IIIb can be converted to the corresponding compound having the formula

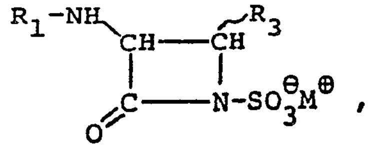

IX

wherein $M^{\oplus}$ is hydrogen or a cation, using the procedures described in United Kingdom patent application 2,071,650. As described therein, a sulfo substituent (—$SO_3^{\ominus}M^{\oplus}$) can be added to the 1-position of an azetidin-2-one by treatment of the azetidin-2-one with a complex of pyridine, 2,6-lutidine or dimethylformamide and sulfur trioxide. An alternative procedure described by the United Kingdom patent comprises silylating an azetidin-2-one (unsubstituted in the 1-position) and then subjecting the silated compound to a silyl interchange reaction. Exemplary silylating agents are monosilyltrifluoroacetamide, trimethylsilylchloride/triethylamine, and bis-trimethylsilyltrifluoroacetamide, and an exemplary reagent useful for the silyl interchange reaction is trimethylsilyl chlorosulfonate.

A compound of formula IX can be converted to the corresponding compound having the formula

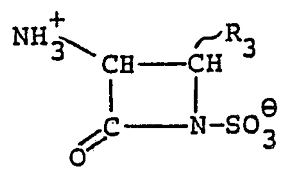

X

by treatment with phosgene followed by treatment with methanol and acid.

Using conventional acylation techniques, a compound of formula X can be converted to the corresponding compound having the formula

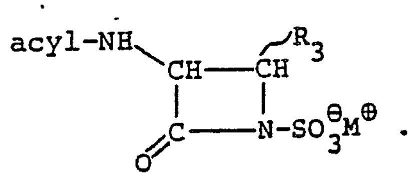

XI .

As described in United Kingdom patent application 2,071,650, a compound of formula X can be reacted with a carboxylic acid, or corresponding carboxylic acid halide or anhydride. The reaction with a carboxylic acid proceeds most readily in the presence of a carbodiimide such as dicyclohexylcarboiimide and a substance capable of forming an active ester *in situ* such as N-hydroxybenzotriazole. In those instances when the acyl group contains reactive functionality (such as amino or carboxyl groups) it may be necessary to first protect those functional groups, then carry out the acylation reaction, and finally deprotect the resulting product.

The ß-lactam antibiotics of formula XI can be used as agents to combat bacterial infections (including urinary tract infections and respiratory infections) in mammalian species, such as domesticated animals and humans. The prior art discloses that for combating bacterial infections in mammals a compound of formula XI can be administered to a mammal in need thereof in an amount of about 1.4 mg/kg/day to about 350 mg/kg/day, preferably about 14 mg/kg/day to about 100 mg/kg/day.

The following examples are specific embodiments of this invention.

Preparation of Starting Materials

(3R-*cis*)-3-Phenylacetylamino-4-norbornylsulfonyl-2-azetidinone

*A)* Penicillin G Sulfoxide

Penicillin G, potassium salt (349.9 g) was dissolved in 3 liters of water. Sodium periodate (194 g) was added and the mixture was stirred for three hours. Dichloromethane (500 ml) was added and the pH of the water layer was adjusted to 2.3 with 6N hydrochloric acid with vigorous stirring. The aqueous layer was separated and extracted with four 400 ml portions of dichloromethane. The combined extract was washed with aqueous sodium bisulfite to remove any iodine color, dried over sodium sulfate, filtered, and evaporated. The solid residue was empasted with 400 ml of ethyl acetate and allowed to stand at 0°C overnight. The solid was isolated by filtration and dried *in vacuo* to affod 322 g of penicillin G sulfoxide.

*B)* Penicillin G Sulfoxide, methyl ester

Penicillin G sulfoxide (321.9 g) and 1000 ml of dichloromethane were cooled in an ice/water bath. A solution of 139.7 g dicyclohexylcarbodiimide in 50 ml of dichloromethane was added followed by a solution of 1.5 g of dimethylaminopyridine in 80 ml of anhydrous methanol. The cold bath was removed and the mixture was stirred for 3.5 hours. The dicyclohexylurea was removed by filtration and 1000 ml of

ethyl acetate was added to the filtrate. The organic layer was washed with sodium bicarbonate solution, water, aqueous sodium dihydrogen phosphate, and water, then dried over sodium sulfate. The solvent was evaporated and the residue was slurried with ethyl acetate to afford 149.6 g of the methyl ester of penicillin G sulfoxide.

*C)* (3R-*cis*)-3-Phenylacetylamino-4-norbornylsulfonyl-2-azetidinone

Finely ground penicillin G sulfoxide methyl ester (25 g) was added in small portions to 250 ml of hot norbornylene containing 6 ml of dioxane. The mixture was refluxed for 16 hours, then most of the excess norbornylene was removed by distillation at 1 atmosphere. Toluene (200 ml) was added and the mixture was evaporated *in vacuo*. The residue was dissolved in 50 ml of dichloromethane and 50 ml of triethylamine was added. After 30 minutes, the mixture was evaporated and chased with toluene. The resulting dark oil was dissolved in 300 ml of dimethylformamide, 80 ml of acetic acid, and 50 ml of water in a 2000 ml flask equipped with a mechanical stirrer. Powdered potassium permanganate (40 g) was added in portions over 20 minutes with cooling in an ice/acetone bath (the temperature was maintained below −5°C). After another 40 minutes 500 ml of ethyl acetate and 500 ml of water were added. Sodium sulfite was added slowly until all of the brown manganese dioxide was dissolved. Additional ethyl acetate was added and the organic phase was washed four times with water, then with sodium bicarbonate solution, and then with saturated brine. The organic layer was dried over sodium sulfate, the ethyl acetate was evaporated and the residue was crystallized from 20 ml of chloroform plus 150 ml of diethyl ether to give 8.2 g of (3R-*cis*)-3-phenylacetylamino-4-norbornylsulfonyl-2-azetidinone.

(3R-*cis* and *trans*)-3-Phenoxyacetylamino-4-phenylsulfonyl-2-azetidinone

*A)* (3R-*cis* and *trans*)-3-Triphenylmethyl-4-phenylsulfonyl-2-azetidinone

A mixture of 30 g of (3R-*cis*)-3-triphenylmethyl-4-phenylsulfonyl-2-azetidinone, 40 g of sodium benzenesulfinate, 25 g of *tetra*-n-butylammonium bromide, 400 ml of 1,2-dichloroethane, and 100 ml of water were refluxed under nitrogen for 30 minutes. The dichloroethane was removed *in vacuo* and the residue was extracted with 700 ml of ethyl acetate. The extract was washed with saturated aqueous sodium bicarbonate solution, then water, then saturated aqueous sodium chloride solution. The extract was dried over sodium sulfate evaporate. The residue was chromatographed on a 50 × 280 mm silica gel column eluted with 1000 ml 1:4 ethyl acetate:hexane, then 1000 ml 1:1 ethyl acetate:hexane. (3R)-3-triphenylmethyl-4-phenylsulfonyl-2-azetidinone (25.3 g) was obtained as a mixture of *cis* and *trans* isomers.

*B)* (3R-*cis* and *trans*)-3-Amino-4-phenylsulfonyl-2-azetidinone hydrochloride

(3R-*cis* and *trans*)-3-triphenylmethyl-4-phenylsulfonyl-2-azetidinone (20.3 g) was dissolved in 200 ml of acetone. Hydrochloric acid (7.2 ml, 12N) was added with stirring. After 2.5 hours, the resulting solid was isolated by filtration, washed with acetone, and dried *in vacuo* to afford 7.6 g of (3R-*cis* and *trans*)-3-amino-4-phenylsulfonyl-2-azetidinone, hydrochloride.

*C)* (3R-*cis* and *trans*)-3-Phenoxyacetylamino-4-phenylsulfonyl-2-azetidinone

To an ice-cooled mixture of 7.6 g of 3-amino-4-phenylsulfonyl-2-azetidinone (mixture of *cis* and *trans* isomers), 5.1 g of sodium bicarbonate 100 ml of dichloromethane, and 50 ml of water was added dropwise with vigorous stirring 4.0 ml phenoxyacetyl chloride. After 90 minutes the resulting solid was removed by filtration and washed with water and dichloromethane. The solid was dissolved in tetrahydrofuran and precipitated with toluene to give 3.85 g of (3R-*trans)-2-phenoxyacetylamino-4-phenylsulfonyl-2-azetidinone, melting point 192—193°C, dec.*

The reaction mixture filtrate was diluted with dichloromethane, washed with water, dried over sodium sulfate, and evaporated to give 4.7 g of a residue which contained both *cis* and *trans* isomers. The residue was triturated with 150 ml of hot chloroform, let stand for 2 hours at 25°C, and then filtered to give 1.2 g of the *trans* isomer. The mother liquor was evaporated and taken up in hot methanol from which 1.35 g of (3R-*cis*-3-phenoxyacetylamino-4-phenylsulfonyl-2-azetidinone, melting point 178—180°C (*dec*), crystallized.

(3R-*cis* and *trans*)-3-Phenylacetylamino-4-phenylsulfonyl-2-azetidinone

The title compound is prepared using the procedure described above for the preparation of the analogous 3-phenoxyacetylamino compound; phenylacetyl chloride is substituted for phenoxy acetyl chloride in part C of the procedure.

(3R-*cis*)-3-Phenoxyacetylamino-4-norbornylsulfonyl-2-azetidinone

The title compound is prepared using the procedure described above for the preparation of the analogous 3-phenoxyacetylamino compound; penicillin V, potassium salt is substituted for penicillin G, potassium salt in part A of the procedure.

Processes for Preparing (S)-3-Acylamino-4-Substituted-2-Azetidinones

(*cis*) and (*trans*)-3-Phenoxyacetylamino-4-methyl-2-azetidinone

Methyl magnesium chloride (2.9 ml, of 2.9 M in tetrahydrofuran) was added to a solution of 500 mg (3R-*trans*)-3-phenoxyacetylamino-4-phenylsulfonyl-2-azetidinone in 11.1 ml of 0.5 M magnesium

dichloride in tetrahydrofuran under nitrogen and chilled in an ice/acetone bath (−10°C). After 2 hours, the mixture was added to saturated aqueous ammonium chloride and extracted with ethyl acetate. The extract was washed with water, dried over sodium sulfate, and evaporated. Treatment of the residue with dichloromethane/ethyl ether gave 106 mg of *cis*-3-phenoxyacetylamino-4-methyl-2-azetidinone. The corresponding *trans* isomer, as well as some *cis* isomer, was present in the mother liquor, as shown by NMR,

(*cis*) and (*trans*)-3-Phenylacetylamino-4-methyl-2-azetidinone

Method I

To 500 mg (3R-*trans*)-3-phenylacetylamino-4-phenylsulfonyl-2-azetidinone in 20 ml tetrahydrofuran under nitrogen and cooled in ice/acetone (−18°C) was added 2.5 ml of 2.9 M methyl magnesium chloride in tetrahydrofuran. After 3.5 hours, the bath temperature had risen to −5°C; the reaction mixture was then added to saturated aqueous ammonium chloride. The mixture was extracted twice with dichloromethane. The combined extract was dried over sodium sulfate, filtered, and evaporated *in vacuo* to give 300 mg residue. NMR indicated an approximate ratio of 15:85 *trans:cis*-3-phenylacetyl-4-methyl-2-azetidinone. The product was dissolved in 2 ml chloroform and precipitated with 3 ml ethyl ether to give 184 mg *cis*-3-phenylacetylamino-4-methyl-2-azetidinone.

Method II

Methyl magnesium chloride (3.0 ml of 2.9 M in tetrahydrofuran) was added to 500 mg (3R-*trans*)-phenylacetylamino-4-phenylsulfonyl-2-azetidinone dissolved in 11.6 ml of 0.5 M magnesium chloride in tetrahydrofuran under nitrogen and chilled in an ice/acetone bath to −10°C; the reaction mixture was poured into saturated aqueous ammonium chloride. The mixture was extracted with ethyl acetate. The extract was washed with water, dried over sodium sulfate, and evaporated. The residue was treated with dichloromethane/ethyl ether to afford 126 mg *cis*-3-phenylacetylamino-4-methyl-2-azetidinone. As shown by NMR, the mother liquor contained the corresponding *trans* isomer as well as some *cis* isomer.

Method III

A solution of methylmagnesium chloride (2.9 m in tetrahydrofuran, 2.2 ml, 6.36 mM) was added to 384 mg (1.06 mM) (3R-*cis*)-3-phenylacetylamino-4-norbornylsulfonyl-2-azetidinone dissolved in 8.5 ml of 0.5 M magnesium chloride in tetrahydrofuran (prepared by the reaction of 2 ml, 1,2-dichloroethane with 0.73 g magnesium in 50 ml of tetrahydrofuran) at 0°C in an ice water bath. The bath as allowed to warm to room temperature over 90 minutes. After another 60 minutes, the reaction mixture was poured into saturated aqueous ammonium chloride. The mixture was extracted with ethyl acetate; the extract was washed with water, dried and evaporated. The residue was chromatographed on a silica gel column with ethyl acetate/hexane, to afford 3-phenylacetylamino-4-methyl-2-azetidinone as a 1:2 mixture of *cis* and *trans* isomers, respectively.

(*cis*) and (*trans*)-3-Phenylacetylamino-4-ethyl-2-azetidinone

To 420 mg (3R-*trans*)-3-phenylacetylamino-4-phenylsulfonyl-2-azetidinone in 20 ml tetrahydrofuran under nitrogen and cooled in an ice/acetone bath to −10°C was added 3.8 ml of 2.08 M ethyl magnesium chloride in tetrahydrofuran. After 4.5 hours, the bath temperature had risen to 0°C; the mixture was added to saturated aqueous ammonium chloride and extracted twice with dichloromethane. The combined extract was washed with water, dried over sodium sulfate, filtered and evaporated. The residue was chromatographed on a silica gel column eluted with 40% ethyl acetate in dichloromethane to give 103 mg product as a *cis* and *trans* mixture of 3-phenylacetylamino-4-ethyl-2-azetidinone in an approximate 5:2 *cis:trans* ratio.

**Claims**

1. A process for preparing a compound having the formula

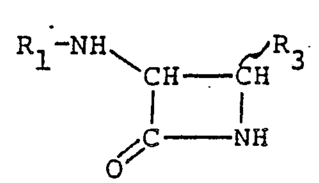

,

which comprises reacting a compound having the formula

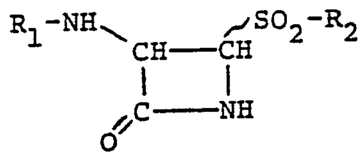

with a Grignard reagent having the formula

$$R_3—Mg\text{-}X_1,$$

wherein

$R_1$ is phenylacetyl or phenoxyacetyl;

$R_2$ is a branched chain alkyl group, phenyl or phenyl substituted with 1, 2 or 3 alkyl (of 1 to 4 carbon atoms) or alkoxy (of 1 to 4 carbon atoms) groups, or norbornyl;

$R_3$ is $C_{1-10}$ alkyl, $C_{2-10}$ alken-1-yl or alkyn-1-yl, 2-phenylethenyl, 2-phenylethynyl, aryl or arylalkyl wherein aryl is phenyl or phenyl substituted with 1, 2 or 3 alkyl (of 1 to 4 carbon atoms) or alkoxy (of 1 to 4 carbon atoms) groups, and

$X_1$ is bromine or chlorine.

2. A process in accordance with claim 1 wherein the reaction is carried out in the presence of a Lewis acid.

3. A process in accordance with claim 2 wherein the Grignard reagent has the formula

$$R_3—Mg\text{-}Cl$$

and the Lewis acid is magnesium chloride.

4. A process in accordance with claim 1 or 3 wherein $R_3$ is methyl.

5. (3R-*cis*)-3-Phenylacetylamino-4-norbornylsulfonyl-2-azetidinone.

6. (3R-*cis*)-3-Phenoxyacetylamino-4-norbornylsulfonyl-2-azetidinone.

**Patentansprüche**

1. Verfahren zur Herstellung einer Verbindung der Formel

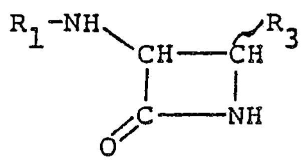

,

dadurch gekennzeichnet, daß man eine Verbindung der Formel

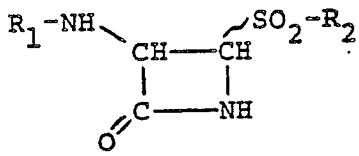

mit einer Grignard-Verbindung der Formel

$$R_3—Mg\text{-}X_1,$$

umsetzt, wobei $R_1$ eine Phenylacetyl- oder eine Phenoxyacetylgruppe ist,

$R_2$ einen Alkylrest mit einer verzweigten Kette, eine Phenylgruppe oder eine mit 1, 2 oder 3 Alkylresten (mit 1 bis 4 Kohlenstoffatomen) oder Alkoxyresten (mit 1 bis 4 Kohlenstoffatomen) substituierte Phenylgruppe oder eine Norbornylgruppe bedeutet,

$R_3$ ein $C_{1-10}$-Alkylrest, eine $C_{2-10}$-Alken-1-yl- oder Alkin-1-ylrest, ein 2-Phenyläthenylrest, ein 2-Phenyläthinylrest, eine Aryl- oder Aralkylrest ist, wobei Aryl eine Phenylgruppe oder eine mit 1, 2 oder 3 Alkylresten (mit 1 bis 4 Kohlenstoffatomen) oder Alkoxyresten (mit 1 bis 4 Kohlenstoffatomen) substituierte Phenylgruppe ist, und

$X_1$ eine Brom- oder Chloratom bedeutet.

2. Verfahren nach Anspruch 1, wobei die Umsetzung in Gegenwart einer Lewis-Säure durchgeführt wird.

3. Verfahren nach Anspruch 2, wobei die Grignard-Verbindung die allgemeine Formel

$$R_3\text{—Mg-Cl}$$

hat und die Lewis-Säure Magnesiumchlorid ist.

4. Verfahren nach Anspruch 1 oder 3, wobei $R_3$ eine Methylgruppe ist.

5. (3R-cis)-3-Phenylacetylamino-4-norbornyl-sulfonyl-2-azetidinon.

6. (3R-cis)-3-Phenoxyacetylamino-4-norbornyl-sulfonyl-2-azetidinon.

**Revendications**

1. Procédé de préparation d'un composé de formule

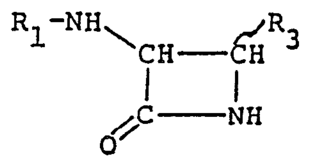

,

qui consiste à faire réagir un composé de formule

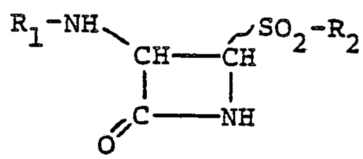

avec un réactif de Grignard ayant pour formule

$$R_3\text{—Mg-}X_1,$$

$R_1$ étant un radical phénylacétyle ou phénoxyacétyle;

$R_2$ étant un radical alkyle à chaîne ramifiée, un radical phényle éventuellement substitué par 1, 2 ou 3 radicaux alkyle (de 1 à 4 atomes de carbone) ou alcoxy (de 1 à 4 atomes de carbone), ou un radical norbornyle;

$R_3$ étant un radical alkyle en $C_{1-10}$, alcén-1-yle ou alcyn-1-yle en $C_{2-10}$, 2-phényléthényle, 2-phényléthynyle, aryle ou arylalkyle, le radical aryle étant un radical phényle éventuellement substitué par 1, 2 ou 3 radicaux alkyle (de 1 à 4 atomes de carbone) ou alcoxy (de 1 à 4 atomes de carbone), et

X étant un atome de brome ou de chlore.

2. Procédé selon la revendication 1, dans lequel la réaction est effectuée en présence d'un acide de Lewis..

3. Procédé selon la revendication 2, dans lequel le réactif de Grignard a pour formule

$$R_3\text{—Mg-Cl}$$

et l'acide de Lewis est le chlorure de magnésium.

4. Procédé selon la revendication 1 ou 3, dans lequel $R_3$ est le radical méthyle.

5. La (3R-cis)-3-phénylacétylamino-4-norbornyl-sulfonyl-2-azétidinone.

6. La (3R-cis)-3-phénoxyacétylamino-4-norbornyl-sulfonyl-2-azétidinone.